# EUROPEAN PATENT APPLICATION

(11) **EP 4 024 047 A1**
(43) Date of publication of application: **06.07.2022**
(21) Application number: 20217775.4
(22) Date of filing: 30.12.2020
(51) Int. Cl.: G01N 33/03, G06Q 10/04, G06Q 10/08

(54) **A METHOD AND A SYSTEM FOR THE DETERMINATION OF THE OVERALL QUANTITY OF USED COOKING OIL PRODUCED BY A PRODUCER**

(30) Priority: 29.12.2020 PT 2020116987
(71) Applicant: Hardlevel - Energias Renováveis, S.A., 4405-516 Serzedo Vng (PT)
(72) Inventor: KARMALI, SALIM, 4405-516 SERZEDO VNG (PT)
(74) Representative: do Nascimento Gomes, Rui

(57) **Abstract**

The present invention is enclosed in the area of used cooking oil waste management, in particular to the automation of used cooking oil waste containers. It is therefore an object of the present invention a method for the determination of the overall quantity of used cooking oil produced by a producer (100), the method comprising the steps of receiving a request message from a communication device of a producer, containing information on a quantity of used cooking oil to be deposited in a used cooking oil container (110), detecting the provision of used cooking oil inside the container (120), and associating the information on a quantity of used cooking oil contained in the request message with a profile of the producer (130), and thereby obtaining an overall quantity of used cooking oil produced by such producer. The method therefore provides a reliable manner to - autonomously - determine a quantity of used cooking oil produced by a producer.

## Description

### FIELD OF THE INVENTION

The present invention is enclosed in the area of used cooking oil waste management, in particular to the automation of used cooking oil waste containers.

### PRIOR ART

In the domestic sector, the destination of the vast majority of used cooking oils is the sewage network, a practice that is highly damaging from the environmental point of view, causing (i) corrosion of the pipes, (ii) the clogging of waste water pipes, (iii) the increase in the load of oils and fats in waste water treatment plants (WWTP), making the treatment of degreasing effluents more costly, since it implies a significant increase in energy consumption and frequency of maintenance and cleaning operations for oil and fat separation equipment, which, are the least to say cumbersome, expensive and environmentally risky.

One litre of used cooking oil dumped into a sewage network can contaminate up to 1,000,000 litres of water, which demonstrates the scale of this problem and reveals the urgency of implementing measures and initiatives that ensure, in an effective manner, an adequate final destination for this particular type of waste, while making the general population aware of the need to adopt best practices in terms of used cooking oil management, promoting a more active involvement of citizens in the correct separation and disposal of this waste.

Used cooking oil waste collection includes the provision of used cooking oil in a centralised location, from which it is collected by the competent services. The solutions known in the art and more commonly used for the purpose of used cooking oil waste collection consist of a large but simple container, in plastic or metal, with non-tamper-evident opening which, in most cases, does not comply with applicable guidelines (such as European Union Guidelines) in terms of characteristics (i) anti-spillage, (ii) fireproof and (iii) anti-theft.

In addition, it should be noted that this sort of equipment only provides the deposit of used cooking oil, and does not allow the control and recording of the amount of used cooking oil deposited, which not only makes it difficult to compile statistical information in a reliable and accurate manner about the amount of used cooking oil collected and sent for later recovery. It also does not allow the planning of the used cooking oil collection routes efficiently and only at the limit of the capacity of the container.

Finally, the systems known in the art do not allow to determine an overall quantity, in litres, of each producer. A producer may be a single person living in a household or the aggregate of a group of persons living in a same household.

In another aspect, the systems known in the art do not offer any type of incentive or compensation to producers, particularly those in the domestic sector, to continuously carry out the correct disposal of used cooking oil waste.

The present invention innovatively overcomes such issues.

### SUMMARY OF THE INVENTION

It is therefore an object of the present invention a method for the determination of the overall quantity of used cooking oil produced by a producer, the method comprising the steps of:
i) in wireless communication means associated with a used cooking oil urban waste container, receiving a request message from a communication device of a producer, the request message containing information on a quantity of used cooking oil to be deposited in a used cooking oil container,
ii) detecting through detection means the provision of used cooking oil or a bottle containing used cooking oil inside the used cooking oil urban waste container,
iii) associating through processing means the information on a quantity of used cooking oil contained in the request message with a profile of the producer, and thereby obtaining an overall quantity of used cooking oil produced by such producer.

The method of the present invention thereby involves that a request message is sent by a communication device of a producer, such as a smartphone, to the wireless communication means, which may be provided in an apparatus associated with the container. The expression associated with may mean on top, inside or provided adjacently to (a few centimetres to some meters next to) the respective container. The container is typically a large size container, which may contain thousands of litres of used cooking oil. The message contains information which is provided by the producer on the quantity of used cooking oil to be deposited in the used cooking oil container. In step ii), detection means, which may be provided in the same apparatus, detect that, in fact, the used cooking oil or a bottle containing used cooking oil has been provided inside the used cooking oil urban waste container. Finally, in step iii), processing means (which may also be provided in the apparatus or in another device) associate the information on a quantity present in the received message with a profile of the producer, and thus obtain an overall quantity of used cooking oil associated with such producer. Such association is performed only if, previously, there has been a detection of a provision of used cooking oil / bottle. The processing means are computational means, which may be complemented by further communication means allowing to communicate with other devices, locally or remotely. The method therefore provides a reliable manner to - autonomously - determine a quantity of used cooking oil produced by a producer. Otherwise, for instance through a determination system which would merely resort to the indication by the user of the quantity, the determined value could be distorted. In addition, it would not allow to automatically associate the quantity with the container. In an aspect, the association of the information on a quantity of used cooking oil contained in the request message with the profile of the producer is dependent of detecting the provision of used cooking oil or a bottle containing used cooking oil inside the used cooking oil urban waste container.

The profile of the user may contain several types of information, besides the overall quantity of used cooking oil that the producer has produced. It may also contain information on the location of a container and associated quantity of a single disposal of used cooking oil / bottle. It may also contain information on an associated household, in particular of the number of sub-producers also associated with such household. It may also contain information on the time and date of each disposal, among other relevant information.

In an aspect, the method for the determination of the overall quantity of used cooking oil produced by a producer of the present invention further comprises the determination of the quantity of used cooking oil and/or bottles containing used cooking oil inside the container.

In an aspect, a system is also an object of the present invention. Such system provides for the determination of the overall quantity of used cooking oil produced by a producer, and comprises:
- an apparatus, the apparatus comprising:
- wireless communication means associated with a used cooking oil urban waste container, the wireless communicating means being configured to receive a request message from a communication device of a producer, the request message containing information on a quantity of used cooking oil to be deposited in a used cooking oil container,
- detection means configured to detect the provision of used cooking oil or a bottle containing used cooking oil inside the used cooking oil urban waste container,
the system being further comprising processing means configured to associate the information on a quantity of used cooking oil contained in the request message with a profile of the producer, and thereby obtaining an overall quantity of used cooking oil produced by such producer and, optionally:
- the apparatus comprises the processing means, or
- the system further comprises a centralized server configured to communicate with the apparatus, the centralized server comprising the processing means.

In an aspect, such system is configured to implement the method for the determination of the overall quantity of the present invention, in any of its embodiments.

In another aspect, another method is also an object of the present invention. Such is a method for the management of a used cooking oil waste collection network which comprises determining the quantity of used cooking oil and/or bottles containing used cooking oil inside a plurality of containers according to the method for the determination of the overall quantity of the present invention, in particular comprising said determination of the quantity inside the container. Such method for the management of a collection network further comprises determining one or more used cooking oil waste collection routes to be performed by one or more a used cooking oil waste collection vehicles based on said quantities associated with said plurality of containers, the one or more routes comprising travel routes to be performed by the vehicles, which include the positions of one or more containers from the plurality of containers. Such method allows to efficiently plan used cooking oil collection routes, and only at the limit of the capacity of each container.

In yet another aspect, another system is also an object of the present invention. Such is a system for the management of a used cooking oil waste collection network which comprises the previously referred system, in any of its embodiments. The system for the management of a used cooking oil waste collection network is further configured to implement the method for the management of a used cooking oil waste collection network, in any of its embodiments.

### DESCRIPTION OF FIGURES

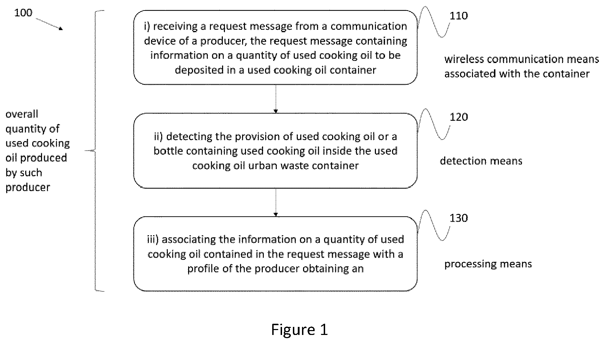

Figure 1 - representation of an embodiment of the method for the determination of the overall quantity of used cooking oil produced by a producer (100) of the present invention, the method comprising the steps of:
   i) in wireless communication means associated with a used cooking oil urban waste container, receiving a request message from a communication device of a producer, the request message containing information on a quantity of used cooking oil to be deposited in a used cooking oil container (110),
   ii) detecting through detection means the provision of used cooking oil or a bottle containing used cooking oil inside the used cooking oil urban waste container (120),
   iii) associating through processing means the information on a quantity of used cooking oil contained in the request message with a profile of the producer (130), and thereby obtaining an overall quantity of used cooking oil produced by such producer.

### DETAILED DESCRIPTION

The more general and advantageous configurations of the present invention are described in the Summary of the invention. Such configurations are detailed below in accordance with other advantageous and/or preferred embodiments of implementation of the present invention.

Several embodiments in respect of the detection of step ii) are subsequently described.

In an inventive aspect, the detection of step ii) further comprises the steps of:
- determining the quantity of used cooking oil and/or bottles containing used cooking oil inside the container prior to the receipt of the message of step i),
- determining a change in the quantity of used cooking oil and/or bottles containing used cooking oil inside the container after the receipt of the message of step i),
- thereby determining that used cooking oil or a bottle containing used cooking oil has been provided inside the container.

Such allows to more reliably detect that used cooking oil or one or more bottles containing used cooking oil have been provided in the container, by determining a change in the quantity. Advantageously, the determination of quantity comprises a determination of the level and/or of the weight of used cooking oil and/or bottles containing used cooking oil inside the container. The determination of level and/or weight provides efficient physical parameters for said detection. In an alternative or cumulative manner, the detection may be performed by an optical sensor, which senses a passage of used cooking oil or one or more bottles containing used cooking oil through an inlet of the container or a section adjacent to the inlet.

In another aspect, the determination of quantity comprises a determination of the level of used cooking oil and/or bottles containing used cooking oil inside the container, the determination of the level comprising the steps of:
- emitting at least one ultrasound signal through ultrasound means comprised by the detection means from a position inside the container which is substantially opposite to a bottom surface of the container, the ultrasound signal being emitted with a direction substantially pointing to and perpendicular to the referred bottom surface,
- receiving an echo signal in the referred ultrasound means,
- identifying in the processing means that the echo signal corresponds to the echo of the emitted at least one ultrasound signal,
- determining through the detection means the time difference between the emission of the ultrasound signal and the reception of the echo signal and, based on such time difference, determining the distance to a surface of used cooking oil or bottles containing used cooking oil provided inside the container, and from such distance determining the level.

In another aspect, the method of the present invention may further comprise the determination of the quantity of used cooking oil or bottles containing used cooking oil provided inside a container. In such case, the quantity may be determined through a level. The determination of the level comprises the steps of:
- emitting at least one ultrasound signal through ultrasound means comprised by the detection means from a position inside the container which is substantially opposite to a bottom surface of the container, the ultrasound signal being emitted with a direction substantially pointing to and perpendicular to the referred bottom surface,
- receiving an echo signal in the referred ultrasound means,
- identifying in the processing means that the echo signal corresponds to the echo of the emitted at least one ultrasound signal,
- determining through the detection means the time difference between the emission of the ultrasound signal and the reception of the echo signal and, based on such time difference, determining the distance to a surface of used cooking oil or bottles containing used cooking oil provided inside the container, and from such distance determining the level.

Advantageously, the emitted ultrasound signal comprises a feature, the feature preferably consisting of one or more predefined frequencies and/or one or more predefined waveforms. Such allows to filter noise possibly present in such context, namely through reflections.

In another aspect of the method of present invention, the container comprises a cover, the cover being configured to cover an inlet of the container, and wherein the method further comprises activating the wireless communication means upon the detection of an opening of the cover, the wireless communication means being otherwise deactivated and, optionally, the detection of an opening of the cover is provided by an electric switch that has a normally closed or open contact, the electric switch being configured to respectively switch the contact to open or closed upon the opening of the cover. Such allows not only to save energy, particularly in a case in which at least the wireless communication are powered by a battery, but also to efficiently initiate a communication with the mobile device of a user, wirelessly.

Preferably, the wireless communication means are local area network wireless communication means, preferably communicating in a frequency in the 2.4 or 5 GHz range, more preferably being configured to communicate via an 802.15 standard, even more preferably through the 802.15.1 standard.

In another aspect of the present invention, the profile of the user further comprises an association of the producer with a household and information on a number of sub-producers in a household, and wherein the overall quantity of used cooking oil produced by the producer is divided by the number of sub-producers, thereby determining an overall quantity of used cooking oil produced by each sub-producer. Such allows to efficiently determine the overall quantity of used cooking oil produced by each sub-producer.

In yet another aspect of the present invention, the profile of the user further comprises an association of the producer with a household, and an undifferentiated urban waste tariff of said household is determined based on the determined overall quantity of used cooking oil of the associated producer. It thereby provides an efficient and automatic methodology for dynamically adapting a value associated with the household, such as the undifferentiated urban waste tariff.

The apparatus of the present invention, associated with a container, may communicate information to a remote location, such as a server, for instance the centralized server. The information may comprise its location and the quantity of used cooking oil and/or bottles contained in it. Such communication may be provided via GPRS, through 2G or NB-loT.

As will be clear to one skilled in the art, the present invention should not be limited to the embodiments described herein, and a number of changes are possible which remain within the scope of the present invention.

Of course, the preferred embodiments shown above are combinable, in the different possible forms, being herein avoided the repetition all such combinations.

## Claims

1. A method for the determination of the overall quantity of used cooking oil produced by a producer (100) **characterised in that** it comprises the steps of:
i) in wireless communication means associated with a used cooking oil urban waste container, receiving a request message from a communication device of a producer, the request message containing information on a quantity of used cooking oil to be deposited in a used cooking oil container (110),
ii) detecting through detection means the provision of used cooking oil or a bottle containing used cooking oil inside the used cooking oil urban waste container (120),
iii) associating through processing means the information on a quantity of used cooking oil contained in the request message with a profile of the producer, and thereby obtaining an overall quantity of used cooking oil produced by such producer (130).

2. A method according to the previous claim wherein the association of the information on a quantity of used cooking oil contained in the request message with the profile of the producer is dependent of detecting the provision of used cooking oil or a bottle containing used cooking oil inside the used cooking oil urban waste container.

3. A method according to any of the preceding claims wherein the detection of step ii) further comprises the steps of:
- determining the quantity of used cooking oil and/or bottles containing used cooking oil inside the container prior to the receipt of the message of step i),
- determining a change in the quantity of used cooking oil and/or bottles containing used cooking oil inside the container after the receipt of the message of step i),
- thereby determining that used cooking oil or a bottle containing used cooking oil has been provided inside the container.

4. A method according to the previous claim wherein the determination of quantity comprises a determination of the level and/or of the weight of used cooking oil and/or bottles containing used cooking oil inside the container.

5. A method according to the previous claim wherein the determination of quantity comprises a determination of the level of used cooking oil and/or bottles containing used cooking oil inside the container, the determination of the level comprising the steps of:
- emitting at least one ultrasound signal through ultrasound means comprised by the detection means from a position inside the container which is substantially opposite to a bottom surface of the container, the ultrasound signal being emitted with a direction substantially pointing to and perpendicular to the referred bottom surface,
- receiving an echo signal in the referred ultrasound means,
- identifying in the processing means that the echo signal corresponds to the echo of the emitted at least one ultrasound signal,
- determining through the detection means the time difference between the emission of the ultrasound signal and the reception of the echo signal and, based on such time difference, determining the distance to a surface of used cooking oil or bottles containing used cooking oil provided inside the container, and from such distance determining the level.

6. A method according to the previous claim wherein the emitted ultrasound signal comprises a feature, the feature preferably consisting of one or more predefined frequencies and/or one or more predefined waveforms.

7. A method according to any of the preceding claims wherein the container comprises a cover, the cover being configured to cover an inlet of the container, and wherein the method further comprises activating the wireless communication means upon the detection of an opening of the cover, the wireless communication means being otherwise deactivated and, optionally, the detection of an opening of the cover is provided by an electric switch that has a normally closed or open contact, the electric switch being configured to respectively switch the contact to open or closed upon the opening of the cover.

8. A method according to any of the preceding claims wherein the wireless communication means are local area network wireless communication means, preferably communicating in a frequency in the 2.4 or 5 GHz range, more preferably being configured to communicate via an 802.15 standard, even more preferably through the 802.15.1 standard.

9. A method according to any of the preceding claims wherein the profile of the user further comprises an association of the producer with a household and information on a number of sub-producers in a household, and wherein the overall quantity of used cooking oil produced by the producer is divided by the number of sub-producers, thereby determining an overall quantity of used cooking oil produced by each sub-producer.

10. A method according to any of the preceding claims wherein the profile of the user further comprises an association of the producer with a household, and an undifferentiated urban waste tariff of said household is determined based on the determined overall quantity of used cooking oil of the associated producer.

11. A method according to any of the preceding claims wherein it comprises determination of the quantity of used cooking oil and/or bottles containing used cooking oil inside the container.

12. A method for the management of a used cooking oil waste collection network **characterised in that** it comprises determining the quantity of used cooking oil and/or bottles containing used cooking oil inside a plurality of containers according to the method of claim 11, and determining one or more used cooking oil waste collection routes to be performed by one or more a used cooking oil waste collection vehicles based on said quantities associated with said plurality of containers, the one or more routes comprising travel routes to be performed by the vehicles, which include the positions of one or more containers from the plurality of containers.

13. A system for the determination of the overall quantity of used cooking oil produced by a producer **characterised in that** it comprises:
- an apparatus, the apparatus comprising:
- wireless communication means associated with a used cooking oil urban waste container, the wireless communicating means being configured to receive a request message from a communication device of a producer, the request message containing information on a quantity of used cooking oil to be deposited in a used cooking oil container,
- detection means configured to detect the provision of used cooking oil or a bottle containing used cooking oil inside the used cooking oil urban waste container,
the system being further comprising processing means configured to associate the information on a quantity of used cooking oil contained in the request message with a profile of the producer, and thereby obtaining an overall quantity of used cooking oil produced by such producer and, optionally:
- the apparatus comprises the processing means, or
- the system further comprises a centralized server configured to communicate with the apparatus, the centralized server comprising the processing means.

14. A system according to the previous claim wherein it is configured to implement the method of any of the claims 1-11.

15. A system for the management of a used cooking oil waste collection network **characterised in that** it comprises the system of any of the claims 13-14 and is configured to implement the method of claim 12.
